# EUROPEAN PATENT SPECIFICATION

(11) **EP 1 993 617 B1**
(45) Date of publication and mention of the grant of the patent: **09.07.2014**
(21) Application number: 07717074.4
(22) Date of filing: 25.01.2007
(51) Int. Cl.: C04B 41/63, C04B 28/02, E04D 1/04, A61L 2/235, A61L 2/232

(54) **BIOCIDAL CONSTRUCTION ARTICLE**
BAUELEMENT MIT BIOZIDEN EIGENSCHAFTEN
ARTICLE DE CONSTRUCTION BIOCIDE

(30) Priority: 14.03.2006 US 375644
(43) Date of publication of application: 26.11.2008
(73) Proprietor: 3M Innovative Properties Company, St. Paul, MN 55133-3427 (US)
(72) Inventor: ANDERSON, Mark T., Saint Paul, Minnesota 55133-3427 (US); GOULD, Rachael A. T., Saint Paul, Minnesota 55133-3427 (US); JACOBS, Jeffry L., Saint Paul, Minnesota 55133-3427 (US)
(74) Representative: Vossius & Partner
(86) International application number: PCT/US2007/002237
(87) International publication number: WO 2007/106242

(56) References cited:
- EP-A1- 0 482 826
- EP-A1- 1 550 691
- WO-A2-02/32830
- WO-A2-2005/110082
- DE-A1- 10 320 503
- US-A- 4 008 351

## Description

### Background

The present disclosure is directed to biocidal construction articles.

Discoloration of roofing substrates and other building materials due to algae infestation has become especially problematic in recent years. Discoloration has been attributed to the presence of blue-green algae, *Gloeocapsa spp.,* transported through air-borne particles. Additionally, discoloration from other airborne contaminants, such as soot, pollen, tree sap, and grease, contribute to discoloration.

In order to combat the discoloration, photocatalytic materials have been combined with roofing substrates and shingles. One example includes photocatalytic titania, which in the presence of ultraviolet light (sunshine) will photo-oxidize the organic materials causing the discoloration.

Currently, no photocatalytic algae-resistant roof tile products are prevalent on the market. Some products claim to provide microbial protection for up to 7 years, such as those products sold under the tradename DUR-A-SHIELD Antimicrobial Surface Protection (acrylate polymer with an anti-microbial agent), available from Dur-A-Shield International, Inc. (Palm Coast, FL). These products rely on antimicrobial agents that lose effectiveness over time.

The general approach to combat discoloration of roofs is periodic washing. This can be done with a high-power water washer. Also sometimes bleach is used in areas where micro-organism infestation is particularly bad. Having a roof professionally washed is a relatively expensive, short-term approach to algae control. The use of bleach can cause staining of ancillary structures and harm surrounding vegetation.

### Summary

Generally, the present disclosure relates to biocidal construction substrates. The present disclosure more particularly relates to biocidal construction substrates that include a biocidal material within the construction substrate and a biocidal polymeric layer disposed on the construction substrate surface.

In one aspect of the disclosure, a biocidal construction substrate is described. The construction substrate includes a structural layer having a biocidal material and an external surface. A biocidal polymeric coating layer is disposed on the external surface. In many embodiments, the biocidal material in the structural layer includes a photocatalytic material. In many embodiments, the biocidal polymeric coating includes a biocidal material and a polymeric binder such as, for example, a polyacrylate. In some embodiments, the biocidal material in the polymeric coating includes, for example, a photocatalytic material, an organic antimicrobial agent, or a transition metal. In one embodiment, the biocidal polymeric coating includes a biocidal polymer having quaternary ammonium pendent groups.

In another aspect of the disclosure, a biocidal roof tile is disclosed. The biocidal roof tile includes a concrete or clay structural layer having photocatalytic particles. The structural layer has an external surface and a polymeric coating layer is disposed on the external surface. The polymeric coating layer has a thickness in a range from 1 to 100 micrometers. The polymeric coating layer includes a polymeric material and a biocidal material. In many embodiments the polymeric material is a polyacrylate. In some embodiments, the biocidal material in the polymeric coating layer includes, for example, a photocatalytic material, an organic antimicrobial agent, or a transition metal.

These and other aspects of the present application will be apparent from the detailed description below. In no event, however, should the above summaries be construed as limitations on the claimed subject matter, which subject matter is defined solely by the attached claims, as may be amended during prosecution.

### Detailed Description

Generally, the present disclosure relates to biocidal construction articles. The present disclosure more particularly relates to biocidal construction substrates that include a biocidal material within the construction substrate and a biocidal polymeric layer disposed on the construction substrate surface.

The construction substrate is a structural layer that can be any layer useful for construction. For example, the structural layer may be an interior or exterior construction surface. A construction substrate is a surface of something man-made. The structural layer may be horizontal or angled such as, for example a floor, a walkway or a roof, or vertical such as, for example, the walls of a building or siding for a buiding. For the purpose of the present application, the term "vertical" includes all non-zero slopes.

The material forming the structural layer may be internal or external. The structural layer may be porous or dense. Specific examples of structural layers include, for example, concrete, clay, ceramic, natural stone and other non-metals. Additional examples of the structural layer include roofs, for example metal roofs, roofing granules, synthetic roofing materials (e.g. composite and polymeric tiles) and asphalt shingles. The structural layer may also be a wall.

The combination of biocidal material within the structural layer and the biocidal coating on the structural layer unexpectedly provides long-term resistance to staining from bio-organisms or from airborne contaminants. In the presence of UV light, for example from sunlight, the photocatalytic titania in the structural layer and coatings, photo-oxidizes organic materials. For example, it oxidizes materials such as volatile organic compounds, soot, grease, and micro-organisms; all of which can cause unsightly discoloration.

The term "polymer" or "polymeric" will be understood to include polymers, copolymers (e.g., polymers formed using two or more different monomers), oligomers and combinations thereof, as well as polymers, oligomers, or copolymers. Both block and random copolymers are included, unless indicated otherwise.

Unless otherwise indicated, all numbers expressing feature sizes, amounts, and physical properties used in the specification and claims are to be understood as being modified in all instances by the term "about." Accordingly, unless indicated to the contrary, the numerical parameters set forth in the foregoing specification and attached claims are approximations that can vary depending upon the desired properties sought to be obtained by those skilled in the art utilizing the teachings disclosed herein.

Weight percent, wt%, percent by weight, % by weight, and the like are synonyms that refer to the concentration of a substance as the weight of that substance divided by the weight of the composition and multiplied by 100.

The term "adjacent" refers to one element being in close proximity to another element and includes the elements touching one another and further includes the elements being separated by one or more layers disposed between the elements.

The term "biocidal" refers to the ability of any composition to inhibit the growth of or to kill microorganisms such as, for example, bacteria, fungi, mold, and algae.

The term "pendent" refers to moieties covalently bound to a polymer.

The recitation of numerical ranges by endpoints includes all numbers subsumed within that range (e.g. 1 to 5 includes 1, 1.5, 2, 2.75, 3, 3.80, 4, and 5) and any range within that range.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

Biocidal construction structures are described. In many embodiments, the construction structure includes a structural layer having a biocidal material and an external surface. A biocidal polymeric coating layer is disposed on the external surface. In some embodiments, the biocidal construction surface is a biocidal roof tile formed from a concrete or clay material having photocatalytic particles and a biocidal polymeric coating layer is disposed on the roof tile. In many embodiments, the polymeric coating layer has a thickness in a range from 0.5 to 100 micrometers, or in a range from 1 to 100 micrometers or in a range from 10 to 100 micrometers, or in a range from 20 to 50 micrometers, and includes a polymeric material and a biocidal material.

The biocidal polymeric coating layer can be formed by combining a biocidal material with a polymeric binder material or can be formed with a biocidal polymer having quaternary ammonium pendent groups, or a combination of these, as desired. The polymeric binder material can be any useful polymeric binder material. When the substrate is cement, concrete, clay, or ceramic the polymeric material can be any useful polymeric material for preventing efflorescence of the substrate. In many embodiments, the polymeric binder is a polyacrylate. In one embodiment, the polymeric binder is a polymethyl(meth)acrylate.

Biocidal polymers include, for example, polymers having quaternary ammonium pendent groups. One embodiment of a biocidal polymer is disclosed in WO 02/10244. This publication discloses a biocidal polyurethane polymer that includes biocidal quaternary ammonium pendent groups.

Any useful biocidal material can be utilized in the structural substrate or within the polymeric binder material. A partial listing of useful biocides includes inorganic antimicrobial agents, for example a photocatalytic material or a transition metal material, and/or an organic antimicrobial agent, for example, a quaternary ammonium compound.

Suitable biocidal material or antimicrobial agents for use in the structural substrate or within the polymeric binder material include any inorganic or organic antimicrobial agent that is effective for reducing contamination by microorganisms, (e.g. pathogens, algae, mold and mildew). Examples of suitable antimicrobial agents include transition metal ion- containing compounds, (e.g., silver, zinc, copper, gold, tin and platinum-based compounds), fatty acid monoesters, triclosan, peroxides, iodines, quaternary ammonium compounds, complexes thereof (e.g., iodophores), derivatives thereof, and combinations thereof.

Examples of suitable commercially available organic antimicrobial agents include polymeric quaternary ammonium salts such as 2-butenyldimethyl ammonium chloride polymers commercially available under the trade designation "POLYQUAT" from Arch Chemicals, Inc., Norwalk, CT; phenolic compounds such as phenol and its derivatives, parabens, and triclosan, which has the chemical formula 2,4,4'-trichloro-2'-hydroxy diphenyl ether, and is commercially available from Ciba Specialty Chemicals, Tarrytown, NY; poly (iminoimidocarbonylimidocarbonyliminohexamethylene hydrochlorides), commercially available under the trade designation "VANTOCIL P" from Arch Chemicals, Inc., Norwalk, CT; polyhexamethylene biguanides, antimicrobial lipids such as those disclosed in Scholz et al., U.S. Publication No. 2005/0089539, antimicrobial acids (e.g., fatty acids, benzoic acids, and salicylic acids), antimicrobial natural oils (e.g., tea tree oils, and grape fruit seed extracts), and combinations thereof. Additional suitable non-reactive organic antimicrobial agents include organic salts of transition metals (i.e., organometallic antimicrobial agents), such as silver salts (e.g., silver lactate), copper salts (e.g., copper napthenate), zinc salts, and tin salts (e.g., trialkyl tin hydroxides and triaryl tin hydroxides).

A biocidal quaternary ammonium compound is present in the construction substrate and/or the polymeric material in any useful amount, as described above. In many embodiments, the biocidal quaternary ammonium compound is present in the construction substrate and/or the polymeric material in a range from 0.01 to 20 wt%, or from 0.1 to 5 wt%.

Examples of suitable silver-containing compounds include silver sulfate, silver acetate, silver chloride, silver lactate, silver phosphate, silver stearate, silver thiocyanate, silver proteinate, silver carbonate, silver nitrate, silver sulfadiazine, silver alginate, silver nanoparticles, silver-substituted ceramic zeolites, silver complexed with calcium phosphates, silver-copper complexed with calcium phosphates, silver dihydrogen citrates, silver iodines, silver oxides, silver zirconium phosphates, silver-substituted glass, and combinations thereof.

Examples of suitable copper-containing compounds include cuprous oxide, which oxidizes to the cupric (2+) state upon exposure to an aqueous reducing agent. Other useful copper compounds useful as algicides include, for example, cupric bromide, cupric oxide, cupric stearate, cupric sulfate, cupric sulfide, cuprous cyanide, cuprous thiocyannate, cuprous stannate, cupric tungstate, cuprous mercuric iodide, and cuprous silicate, or mixtures thereof.

Transition metal material is present in the construction substrate and/or the polymeric material in any useful amount, as described above. In many embodiments, the transition metal material is present in the construction substrate and/or the polymeric material in a range from 0.1 to 70 vol%, or from 1 to 50 vol%, or from 5 to 40 vol%, or from 10 to 30 vol%. Copper oxides can be present in the construction substrate and/or the polymeric material in a range from 0.1 to 70 vol%, or from 1 to 50 vol%, or from 10 to 30 vol%. Silver can be present in the construction substrate and/or the polymeric material in a range from 0.01 to 50 vol%, or from 0.1 to 10 vol%. Tin silane can be present in the construction substrate and/or the polymeric material in a range from 0.001 to 10 g/m², or from 0.1 to 10 g/m².

Suitable commercially available silver zeolites-containing compounds include those sold under the trade designation "AGION" from AgION Technologies Inc., Wakefield, MA; those available under the trade designations "IRGAGUARD B5000" and "IRGAGUARD B8000", which are based on AgZn zeolites supplied by Ciba Specialty Chemicals, Tarrytown, NY; as well as those available under the trade designation "ALPHASAN", which are silver sodium hydrogen zirconium phosphates, supplied by Milliken Chemicals, Spartanburg, SC. Suitable commercially available silver chloride-containing compounds include those available under the trade designation "JMAC" from Clariant Corporation, Charlotte, NC.

Suitable concentrations of the antimicrobial agents in structural substrate or within the polymeric binder material include any concentration that is effective for reducing microbial contamination. Examples of suitable concentrations of the antimicrobial agents in the structural substrate or within the polymeric binder material range from about 0.1 % by weight to about 20% by weight, with particularly suitable concentrations ranging from about 1% by weight to about 10% by weight.

Photocatalysts, upon activation or exposure to sunlight, establish both oxidation and reduction sites. These sites are capable of preventing or inhibiting the growth of microorganisims such as, for example, algae on the substrate or generating reactive species that inhibit the growth of algae on the substrate. In other embodiments, the sites generate reactive species that inhibit the growth of biota on the substrate. The sites themselves, or the reactive species generated by the sites, may also photooxidize other surface contaminants such as dirt or soot or pollen. Photocatalytic elements are also capable of generating reactive species which react with organic contaminants converting them to materials which volatilize or rinse away readily. Photocatalytic particles conventionally recognized by those skilled in the art are suitable for use with the present invention. Suitable photocatalysts include, but are not limited to, TiO₂, ZnO, WO₃, SnO₂, CaTiO₃, Fe₂O₃, MoO₃, Nb₂O₅, TiₓZr₍₁₋ₓ₎O₂, SiC, SrTiO₃, CdS, GaP, InP, GaAs, BaTiO₃, KnbO₃, Ta₂O₅, Bi₂O₃, NiO, Cu₂O, SiO₂, MoS₂, InPb, RuO₂, CeO₂, Ti(OH)₄, combinations thereof, or inactive particles coated with a photocatalytic coating. In other embodiments, the photocatalytic particles are doped with, for example, carbon, nitrogen, sulfur, fluorine, and the like. In other embodiments, the dopant may be a metallic element such as Pt, Ag, or Cu. In some embodiments, the doping material modified the bandgap of the photocatalytic particle. In some embodiments, the transition metal oxide photocatalyst is nanocrystalline anatase TiO₂.

Relative photocatalytic activities of a substrate, substrate coating and/or coated substrate can be determined via a rapid chemical test that provides an indication of the rate at which hydroxyl radicals are produced by UV-illuminated photocatalyst in or on the substrate. One method to quantify the production of hydroxy radicals produced by a photocatalyst is through use of the 'terephthalate dosimeter' which has been cited numerous times in the open literature. Recent publications include: "Detection of active oxidative species in TiO2 photocatalysts using the fluorescence technique" Ishibashi, K; et. al. Electrochem. Comm. 2 (2000) 207-210. "Quantum yields of active oxidative species formed on TiO2 photocatalyst" Ishibashi, K; et al. J. Photochem. and Photobiol. A: Chemistry 134 (2000) 139-142.

Photocatalytic material is present in the construction substrate and/or the polymeric material in any useful amount, as described above. In many embodiments, the photocatalytic material is present in the construction substrate and/or the polymeric material in a range from 0.1 to 70 vol%, or from 1 to 50 vol%, or from 5 to 40 vol%, or from 10 to 30 vol%.

The following examples further disclose embodiments.

### Examples

### General Approach to making Photocatalytic Tile (cementicious substrate)

Photocatalytic tiles generally contain: Portland cement, sand, water, and a photocatalyst. Photocatalysts include TiO₂, WO₃, ZnO and similar wide-bandgap semiconducting metal oxides. In many embodiments, photocatalysts include the anatase form of TiO₂ and or mixtures of anatase TiO₂ and ZnO (e.g. Catalite 4000). The water-to-cement weight ratio, in many embodiments, is in the range from 0.3 to 1. The cement to sand weight ratio, in many embodiments, is in a range from 0.2 to 1. The photocatalyst content, in many embodiments, is in a range from 0.5 to 75 volume percent, or 5 to 50 volume percent, or 10 to 30 volume percent.

The materials are mixed to uniform consistency and formed into tiles of the desired shape. Biocidal coatings described below can then be formed on these photocatalytic tiles.

### Example 1- Biocidal Coating

A biocide is mixed with a polymer to form a coating solution. The coating solution is applied to a photocatalytic cementitious substrate and allowed to dry. This dried biocide coating has a thickness, in many embodiments, in a range from 10 to 100 micrometers or from 20 to 50 micrometers.

### Example 2 - Photocatalyst and acrylate

One part of a dispersion of photocatalytic particles (e.g. STS-21 40 wt% aqueous sol from Ishihara Corp, San Francisco, CA) is combined with 5 parts of an aqueous polyacrylate emulsion (e.g. Rhoplex available from Rhom and Haas, Philadelphia PA) to form an aqueous coating solution. The solution is sprayed or painted onto a photocatalytic roofing tile and allowed to dry to form a coating that is in a range from 20 to 50 micrometers thick.

### Example 3 - Copper oxide and acrylate

40 g of copper(I) oxide (e.g. from American Chemet, Deerfield, IL) is combined with 0.5 g of surfactant (e.g. sodium lauryl sulfate available from Chemron Corp, Bowling Green, OH) and 60 g water to form an approx 40% w/w aqueous suspension. One part copper oxide suspension is added to one parts of an aqueous polyacrylate emulsion (e.g. Rhoplex available from Rhom and Haas, Philadelphia PA) to form an aqueous coating solution. The resulting solution is shaken vigorously and then applied onto a photocatalytic roofing tile and allowed to dry to form a coating that is in a range from 20 to 50 micrometers thick.

### Example 4 - Tin silane and acrylate

Tin silane is prepared by the following procedure as taught in US 5,415,919. In an atmosphere of dry nitrogen, 99.8 g tributyltin hydride (Lancaster Synthesis, Windham, NH, as supplied) and 71.0 g triethoxyvinylsilane (Petrarch Systems, Bristol, PA, as supplied) were mixed with 0.13 g AIBN catalyst (Aldrich Chemical Co., Milwaukee, WI, as supplied) added in three portions at 0, 3, and 6 hr reaction time. The reaction mixture was heated to 80-85° C. for a total of 23 hr. spectroscopic analysis of the mixture showed the reaction to be complete, and infrared, nuclear magnetic resonance (¹ H and ¹³ C), and mass spectral analysis and elemental analysis confirmed that the product is [2-(triethoxysilyl)ethyl]-tributyltin or (n-Bu)₃ SnCH₂ CH₂ Si(OEt)₃.

One part tin silane from above is combined with 10 parts of an aqueous polyacrylate emulsion (e.g. Rhoplex available from Rhom and Haas, Philadelphia PA) to form an aqueous coating solution. The resulting solution is shaken and then applied onto a photocatalytic roofing tile and allowed to dry to form a coating that provides tin at ∼0.1 g/m².

### Example 5 - Quaternary ammonium salt and acrylate

One part of a commercially available ammonium salt (tri-methoxy silyl propyl dimethyl octadecyl ammonium chloride) in acrylate, available from Aegis Environments (available from Aegis Laboratories, Midland MI; tradename Microbe Shield) is combined with 50 parts of an aqueous polyacrylate emulsion (e.g. Rhoplex available from Rhom and Haas, Philadelphia PA) to form an aqueous coating solution. The resulting solution is shaken and then applied onto a photocatalytic roofing tile and allowed to dry to form a coating that is in a range from 20 to 50 micrometers thick.

### Example 6 - Silver and acrylate

One part of the silver-containing resin AlphaSan (e.g. from Milliken Chemical, Spartanburg, SC) is combined with 20 parts of an aqueous polyacrylate emulsion (e.g. Rhoplex available from Rhom and Haas, Philadelphia PA) to form an aqueous coating solution. The resulting solution is shaken and then applied onto a photocatalytic roofing tile and allowed to dry to form a coating that is in a range from 20 to 50 micrometers thick.

## Claims

1. A construction structure comprising:
a structural layer comprising a biocidal material, the structural layer having an external surface; and
a biocidal polymeric coating layer on the external surface.

2. The construction structure according to claim 1 wherein the structural layer biocidal material comprises photocatalytic particles and the biocidal polymeric coating layer comprises photocatalytic particles and a polymeric material.

3. The construction structure according to claim 1 wherein the structural layer biocidal material comprises photocatalytic particles and the biocidal polymeric coating layer comprises an organic antimicrobial agent and a polymeric material:

4. The structure according to claim 1 wherein the structural layer biocidal material comprises photocatalytic particles and the biocidal polymeric coating layer comprises an inorganic antimicrobial agent and a polymeric material.

5. The construction structure according to claim 1 wherein the structural layer biocidal material comprises photocatalytic particles and the biocidal polymeric coating layer comprises a quaternary ammonium compound and a polymeric material.

6. The construction structure according to claim 1 wherein the structural layer biocidal material comprises photocatalytic particles and the biocidal polymeric coating layer comprises a transition metal containing compound.

7. The construction structure according to claim 1 wherein the structural layer biocidal material comprises photocatalytic particles and the biocidal polymeric coating layer comprises a biocidal polymer having quaternary ammonium pendent groups.

8. The construction structure according to claim 2 wherein the photocatalytic particles comprise TiO₂, ZnO, WO₃, SnO₂, CaTiO₃, Fe₂O₃, MoO₃, Nb₂O₅, TiₓZr₍₁₋ₓ₎O₂, SiC, SrTiO₃, CdS, GaP, InP, GaAs, BaTiO₃, KNbO₃, Ta₂O₅, Bi₂O₃, NiO, Cu₂O, MoS₂, InPb, RuO₂, CeO₂, Ti(OH)₄, or combinations thereof.

9. The construction structure according to claim 2 wherein the polymeric binder comprises a polyacrylate.

10. The construction structure according to claim 1 wherein the exterior surface comprises photocatalytic particles and the biocidal polymeric coating layer comprises photocatalytic particles and a polymeric material.

11. A roof tile comprising:
a concrete or clay structural layer comprising photocatalytic particles, the structural layer having an external surface; and
a polymeric coating layer on the external surface, the polymeric coating layer having a thickness in a range from 1 to 100 micrometers, the polymeric coating layer comprising:
a polymeric material; and
a biocidal material.

12. The roof tile according to claim 11 wherein the biocidal material comprises photocatalytic particles.

13. The roof tile according to claim 11 wherein the biocidal material comprises an inorganic antimicrobial agent.

14. The roof tile according to claim 11 wherein the polymeric material is polyacrylate.

15. The roof tile according to claim 11 wherein the polymeric material is polymethyl(meth)acrylate.

## Patentansprüche

1. Baustruktur, umfassend:
eine Strukturschicht, umfassend ein biozides Material, wobei die Strukturschicht eine Außenoberfläche aufweist; und
eine biozide Polymerbeschichtungsschicht auf der Außenoberfläche.

2. Baustruktur nach Anspruch 1, wobei das biozide Material der Strukturschicht photokatalytische Teilchen umfasst und die biozide Polymerbeschichtungsschicht photokatalytische Teilchen und ein Polymermaterial umfasst.

3. Baustruktur nach Anspruch 1, wobei das biozide Material der Strukturschicht photokatalytische Teilchen umfasst und die biozide Polymerbeschichtungsschicht ein organisches antimikrobielles Mittel und ein Polymermaterial umfasst.

4. Baustruktur nach Anspruch 1, wobei das biozide Material der Strukturschicht photokatalytische Teilchen umfasst und die biozide Polymerbeschichtungsschicht ein anorganisches antimikrobielles Mittel und ein Polymermaterial umfasst.

5. Baustruktur nach Anspruch 1, wobei das biozide Material der Strukturschicht photokatalytische Teilchen umfasst und die biozide Polymerbeschichtungsschicht eine quaternäre Ammoniumverbindung und ein Polymermaterial umfasst.

6. Baustruktur nach Anspruch 1, wobei das biozide Material der Strukturschicht photokatalytische Teilchen umfasst und die biozide Polymerbeschichtungsschicht eine übergangsmetallhaltige Verbindung umfasst.

7. Baustruktur nach Anspruch 1, wobei das biozide Material der Strukturschicht photokatalytische Teilchen umfasst und die biozide Polymerbeschichtungsschicht ein biozides Polymer mit seitenständigen quaternären Ammoniumgruppen umfasst.

8. Baustruktur nach Anspruch 2, wobei die photokatalytischen Teilchen TiO₂, ZnO, WO₃, SnO₂, CaTiO₃, Fe₂O₃, MoO₃, Nb₂O₅, TiₓZr(₁₋ₓ)O₂, SiC, SrTiO₃, CdS, GaP, InP, GaAs, BaTiO₃, KNbO₃, Ta₂O₅, Bi₂O₃, NiO, Cu₂O, MoS₂, InPb, RuO₂, CeO₂, Ti(OH)₄ oder Kombinationen davon umfassen.

9. Baustruktur nach Anspruch 2, wobei das polymere Bindemittel ein Polyacrylat umfasst.

10. Baustruktur nach Anspruch 1, wobei die Außenoberfläche photokatalytische Teilchen umfasst und die biozide Polymerbeschichtungsschicht photokatalytische Teilchen und ein Polymermaterial umfasst.

11. Dachziegel, umfassend:
eine Beton- oder Ton-Strukturschicht, umfassend photokatalytische Teilchen, wobei die Strukturschicht eine Außenoberfläche aufweist; und
eine Polymerbeschichtungsschicht auf der Außenoberfläche, wobei die Polymerbeschichtungsschicht eine Dicke im Bereich von 1 bis 100 Mikrometer aufweist, wobei die Polymerbeschichtungsschicht:
ein Polymermaterial und
ein biozides Material
umfasst.

12. Dachziegel nach Anspruch 11, wobei das biozide Material photokatalytische Teilchen umfasst.

13. Dachziegel nach Anspruch 11, wobei das biozide Material ein anorganisches antimikrobielles Mittel umfasst.

14. Dachziegel nach Anspruch 11, wobei es sich bei dem Polymermaterial um Polyacrylat handelt.

15. Dachziegel nach Anspruch 11, wobei es sich bei dem Polymermaterial um Polymethyl(meth)acrylat handelt.

## Revendications

1. Structure de construction comprenant :
une couche structurale comprenant un matériau biocide, la couche structurale comportant une surface externe ; et
une couche de revêtement polymère biocide sur la surface externe.

2. Structure de construction selon la revendication 1, dans laquelle le matériau biocide de la couche structurale comprend des particules photocatalytiques et la couche de revêtement polymère biocide comprend des particules photocatalytiques et un matériau polymère.

3. Structure de construction selon la revendication 1, dans laquelle le matériau biocide de la couche structurale comprend des particules photocatalytiques et la couche de revêtement polymère biocide comprend un agent antimicrobien organique et un matériau polymère.

4. Structure de construction selon la revendication 1, dans laquelle le matériau biocide de la couche structurale comprend des particules photocatalytiques et la couche de revêtement polymère biocide comprend un agent antimicrobien inorganique et un matériau polymère.

5. Structure de construction selon la revendication 1, dans laquelle le matériau biocide de la couche structurale comprend des particules photocatalytiques et la couche de revêtement polymère biocide comprend un composé d'ammonium quaternaire et un matériau polymère.

6. Structure de construction selon la revendication 1, dans laquelle le matériau biocide de la couche structurale comprend des particules photocatalytiques et la couche de revêtement polymère biocide comprend un composé contenant un métal de transition.

7. Structure de construction selon la revendication 1, dans laquelle le matériau biocide de la couche structurale comprend des particules photocatalytiques et la couche de revêtement polymère biocide comprend un polymère biocide comportant des groupes pendants d'ammonium quaternaire.

8. Structure de construction selon la revendication 2, dans laquelle les particules photocatalytiques comprennent TiO₂, ZnO, WO₃, SnO₂, CaTiO₃, Fe₂O₃, MoO₃, Nb₂O₅, TiₓZr₍₁₋ₓ₎O₂, SiC, SrTiO₃, CdS, GaP, InP, GaAs, BaTiO₃, KNbO₃, Ta₂O₅, Bi₂O₃, NiO, Cu₂O, MoS₂, InPb, RuO₂, CeO₂, Ti(OH)₄, ou des combinaisons de ceux-ci.

9. Structure de construction selon la revendication 2, dans laquelle le liant polymère comprend un polyacrylate.

10. Structure de construction selon la revendication 1, dans laquelle la surface externe comprend des particules photocatalytiques et la couche de revêtement polymère biocide comprend des particules photocatalytiques et un matériau polymère.

11. Tuile de couverture comprenant :
une couche structurale de béton ou d'argile comprenant des particules photocatalytiques, la couche structurale comportant une surface externe ; et
une couche de revêtement polymère sur la surface externe, la couche de revêtement polymère ayant une épaisseur de 1 micromètre à 100 micromètres, la couche de revêtement polymère comprenant :
un matériau polymère ; et
un matériau biocide.

12. Tuile de couverture selon la revendication 11, dans laquelle le matériau biocide comprend des particules photocatalytiques.

13. Tuile de couverture selon la revendication 11, danslaquelle le matériau biocide comprend un agent antimicrobien inorganique.

14. Tuile de couverture selon la revendication 11, dans laquelle le matériau polymère est un polyacrylate.

15. Tuile de couverture selon la revendication 11, dans laquelle le matériau polymère est un poly(méth)acrylate de méthyle.
